(19) Europäisches Patentamt
European Patent Office
Office européen des brevets

(11) **EP 0 764 656 B1**

(12) **EUROPÄISCHE PATENTSCHRIFT**

(45) Veröffentlichungstag und Bekanntmachung des Hinweises auf die Patenterteilung:
**16.06.1999 Patentblatt 1999/24**

(51) Int Cl.$^6$: **C07H 15/04**

(21) Anmeldenummer: **96114364.1**

(22) Anmeldetag: **07.09.1996**

(54) **Verfahren zur Auftrennung von Alkylglycosiden**

Method of separation of alkyl glycosides

Méthode de séparation des alkyl-glycosides

(84) Benannte Vertragsstaaten:
**BE CH DE ES FR GB IT LI NL**

(30) Priorität: **21.09.1995 DE 19535031**

(43) Veröffentlichungstag der Anmeldung:
**26.03.1997 Patentblatt 1997/13**

(73) Patentinhaber: **Th. Goldschmidt AG**
**45127 Essen (DE)**

(72) Erfinder:
* **Grüning, Burghard, Dr.**
**45134 Essen (DE)**
* **Peter, Siegfried, Prof. Dr.**
**91080 Uttenreuth-Weiher (DE)**
* **Weidner, Eckhard, Dr.**
**91056 Erlangen (DE)**
* **Zhang, Zhengfeng, Dr.**
**82327 Tutzing (DE)**

(56) Entgegenhaltungen:
**EP-A- 0 639 551** **EP-A- 0 712 857**
**EP-A- 0 739 975**

* **DATABASE WPI Section Ch, Week 9021 Derwent Publications Ltd., London, GB; Class A11, AN 90-160732 XP002020980 & JP 02 103 202 A (NIPPON CORNSTARCH K), 16.April 1990**
* **DATABASE WPI Section Ch, Week 9213 Derwent Publications Ltd., London, GB; Class D25, AN 92-102825 XP002007147 & JP 04 049 297 A (KAO CORP), 18.Februar 1992**
* **FOOD SCI. TECHNOL. (N. Y.), Bd. 62, 1994, Seiten 65-93, XP002020978 BOUTTE T T ET AL: "Supercritical fluid extraction and chromatography of sucrose and methyl glucose polyester"**
* **CHEMIE-INGENIEUR-TECHNIK, Bd. 68, Nr. 6, Juni 1996, Seiten 717-721, XP002020979 SRIDHAR S: "Entfernung hochsiedender Alkohole durch Extraktion mit Gasen im überkritischen Zustand"**

**Beschreibung**

Die Erfindung betrifft ein Verfahren zur Auftrennung von Alkylglycosidgemischen, bei denen der aus der Synthese stammende überschüssige Alkohol zuvor abgetrennt wurde, durch Extraktion. Als Produkte können z. B. reine Alkylmonoglycoside oder hellfarbige Alkylglycosidgemische gewonnen werden.

Alkylglycoside sind durch Reaktion von reduzierenden Zuckern mit aliphatischen Alkoholen zugänglich. Als Zucker kommen Mono-, Di- und Polysaccharide in Betracht. Beispiele sind Saccharose, Maltose, Galaktose und vorzugsweise Glucose. Diese werden in Gegenwart eines sauren Katalysators bei erhöhten Temperaturen mit aliphatischen Alkoholen mit Kettenlängen bis zu 30 Kohlenstoffatomen umgesetzt. Als Beispiel sei die DE-OS 38 33 780 genannt. Aufgrund der Unverträglichkeit von längerkettigen Alkoholen und Zuckern ist auch ein zweistufiges Umacetalisierungsverfahren entwickelt worden. Im ersten Schritt wird ein kürzerkettiger Alkohol, vorzugsweise Butanol oder Propylenglycol, mit dem Zucker zum Glycosid umgesetzt, das nun in einem weiteren Schritt mit einem Fettalkohol zum längerkettigen Alkylglycosid umglycosidiert wird (Seife, Öle, Fette, Wachse 118, 1992, S. 894 bis 904). Die erhaltenen Gemische bestehen aus Alkyloligoglycosiden mit einer unterschiedlichen Anzahl von Zuckereinheiten und den nicht umgesetzten aliphatischen Alkoholen, die in der Regel im Überschuß eingesetzt werden. Der überschüssige Alkohol kann anschließend destillativ (DE-OS 39 32 173) abgetrennt werden. Durch die Reaktion und vor allem die Destillation wird das Reaktionsgemisch thermisch stark belastet, so daß dunkel gefärbte Nebenprodukte entstehen, die die Qualität des Alkylglycosids beeinträchtigen. Daher sind verschiedene Verfahren zur Bleichung von Alkylglycosiden beschrieben worden. Als Beispiele seien die EP-A-0 526 710, die DE-OS 40 19 175 und die DE-OS 39 40 827 genannt, in denen die Bleichung durch Bestrahlung, durch Zusatz von Wasserstoffperoxid bzw. durch Behandlung mit Ozon durchgeführt wird.

Die EP-A-712 857, relevant gemäß Art. 54(3) EPÜ, beschreibt ein Verfahren, zur Herstellung von Alkylglykosiden und die nachfolgende Abtrennung des überschüssigen Alkohols durch Extraktion.

Die EP-A-739 975, relevant gemäß Art. 54(3) EPÜ, betrifft die Reinigung von Alkylpolyglykosiden, wobei die Verunreinigungen aus dem Polyglycosidgemisch entfernt werden.

Aus den JP-A-2 103 202, JP-A-4 049 297 sowie der EP-A-639 551 ist ebenfalls nur die Reinigung von Alkylglykosiden zu entnehmen, während die Veröffentlichung Food Sci. Technol. (N.Y.), Band 62, pp 65-93 (1994) die Reinigung von Zuckerestern beschreibt.

Die nach der Abtrennung des Alkohols erhaltenen Alkylglycoside stellen ein Gemisch aus unterschiedlichen Anteilen von Mono-, Di-, Tri- und höheren Glycosiden dar. Das Monoglycosid bildet zumeist die Hauptkomponente. Der Oligomerisierungsgrad kann in gewissen Grenzen durch das Molverhältnis der Edukte, des Alkohols und des Zuckers bestimmt werden. So kann durch einen hohen Alkoholüberschuß vorzugsweise Monoglycosid gewonnen werden, während durch einen möglichst geringen Alkoholüberschuß höhere Oligomerisierungsgrade zugänglich sind. Ein niedriger Oligomerisierungsgrad wird so allerdings durch geringe Volumenausbeuten erkauft, während in Reaktionen, die auf höhere Oligomerisierungsgrade zielen, die Viskosität der Reaktionsmischungen sehr hoch sein kann und die Gefahr der Polysaccharidbildung stark zunimmt. Diese Verfahrensvarianten ermöglichen darüber hinaus nicht die Gewinnung reiner oder weitgehend reiner Oligomerer. Eine Auftrennung des Produktgemisches in die einzelnen Bestandteile, wie Mono- und Diglycosid und höhere Oligomerisierungsgrade, ist bislang nicht beschrieben und war technisch nicht möglich. Diese konnte jedoch mit dem Verfahren der vorliegenden Erfindung erzielt werden. Darüber hinaus können mit diesem Verfahren hellfarbige Alkylglycoside ohne Anwendung eines Bleichprozesses erhalten werden.

Gegenstand der vorliegenden Erfindung ist ein Verfahren zur Auftrennung von Alkylglycosidgemischen, bei denen der aus der Synthese stammende überschüssige Alkohol zuvor abgetrennt wurde, durch Extraktion, welches dadurch gekennzeichnet ist, daß die Extraktion mit einem nahekritischen oder überkritischen Extraktionsmittel, das sich im Bereich der reduzierten Temperatur von 0,7 bis 1,3 befindet, und bei Drücken, die so gewählt werden, daß die Dichte des Extraktionsmittels ≥ 180 kg/m³ beträgt, durchgeführt wird. Als Produkte können z. B. reine entfärbte Alkylmonoglycoside oder hellfarbige Alkylpolyglycoside gewonnen werden. Die Alkylpolyglycoside werden in der Regel Gemische aus vorwiegend Monoglycosiden sowie Di-, Triund Tetraglycosiden darstellen, die auch geringere Mengen an höheren Glycosiden enthalten können.

Unter reduzierter Temperatur $T_r$ ist die dimensionslose Größe zu verstehen, die sich durch Division der gemessenen Temperaturen (in Kelvin) durch die kritische Temperatur $T_c$ (Kelvin) des Systems gemäß folgender Formel

$$T_r = \frac{T}{T_c}$$

ergibt.

Vorzugsweise werden als Extraktionsmittel Kohlendioxid, Kohlenwasserstoffe mit 1 bis 10 C-Atomen und Ether mit 2 bis 8 C-Atomen oder Gemische aus diesen verwendet.

Als Cosolventien können den Extraktionsmitteln bis zu 35 % niedermolekulare aliphatische Lösungsmittel und/oder Wasser zugesetzt werden. Als Beispiele für aliphatische Lösungsmittel sind zu nennen aliphatische Alkohole, wie Methanol, Ethanol, Propanol, iso-Propanol, Ketone, wie Aceton, Methylethylketon, Säuren, wie Ameisensäure, Essigsäure. Bevorzugt ist Ethanol. Hierdurch kann die Beladung der Extraktionsmittelphase erhöht werden.

Als Ausgangsprodukte für die Fraktionierung werden Alkylglycosidgemische verwendet, bei denen der aus der Synthese stammende überschüssige Alkohol zuvor abgetrennt wurde. Durch die Extraktion kann das Alkylglycosidgemisch in eine Extraktphase, die die Alkylglycoside mit niedrigem Oligomerisierungsgrad enthält, und in eine Raffinatphase, die die Alkylglycoside mit hohem Oligomerisierungsgrad sowie die farbigen Nebenprodukte und Verunreinigungen enthält, aufgetrennt werden. Durch die Wahl des Extraktionsmittels und der geeigneten Bedingungen, wie z. B. Temperatur und Druck, läßt sich die Zusammensetzung der Extrakt- und Raffinatphase einstellen. So ist es beispielsweise möglich, daß die am Kopf einer Gegenstromkolonne abgenommene Extraktphase als Alkylglycoside ausschließlich Alkylmonoglycoside enthält und die höheren Alkylglycoside in der Raffinatphase verbleiben.

In einem anderen Extremfall können die Alkylglycoside nahezu vollständig in die Extraktphase extrahiert werden, so daß in der Raffinatphase im wesentlichen nur der kleine Anteil der farbigen Nebenprodukte und Verunreinigungen zurückbleibt. Auf diesem Wege wird zusätzlich eine Entfärbung der Alkylglycoside erreicht. Diese beruht jedoch im Unterschied zur Bleichung auf einer Abtrennung der farbigen Verbindungen, wodurch die Qualität des Alkylglycosids verbessert wird.

Durch geeignete Wahl der Bedingungen ist es möglich, zwischen diesen beiden Extremfällen liegende Fraktionen abzutrennen, z. B. eine Fraktion, die überwiegend aus Mono- bis Triglycosiden besteht. Ebenso ist es möglich, falls gewünscht, durch mehrfache Extraktion unter veränderten Bedingungen, sei es in einer aus mehreren Trennkolonnen bestehenden Extraktionsanlage oder durch mehrfache Extraktion auf der gleichen Trennkolonne, ein Alkylglycosidgemisch in mehrere Fraktionen zu zerlegen. Diese weisen dann unterschiedliche Anteile an den jeweiligen Alkyloligoglycosiden auf.

Durch Temperaturerhöhung und/oder Druckerniedrigung werden die Produkte im Extraktionsmittel unlöslich. Die extrahierte Alkylglycosidfraktion kann so in einem Abscheider vom Extraktionsmittel getrennt werden. Durch Versprühen lassen sich letzte Reste des Extraktionsmittels vollständig entfernen, und die Produkte werden in feinverteilter, fester Form erhalten.

Das Verfahren dieser Erfindung liefert u. a. folgende Vorteile:

    a) Es lassen sich reine Alkylmonoglycoside abtrennen und gewinnen.
    b) Es können reine, farblose Alkylmonoglycosid-freie Alkylglycoside gewonnen werden.
    c) Alkylglycoside können durch Abtrennung der farbigen Nebenprodukte und Verunreinigungen entfärbt werden.
    d) Die Extraktion kann kontinuierlich im Gegenstrom durchgeführt werden.
    e) Die Extraktionsmittel können bei niedriger Temperatur vollständig entfernt werden.

Alkylmonoglycoside, vor allem Alkylmonoglucoside, insbesondere solche mit Alkylresten, die sich von Fettalkoholen mit 10 bis 14 Kohlenstoffatomen ableiten, eignen sich als besonders wirksame reinigende Tenside, die vorzugsweise in Tensidgemischen eingesetzt werden, die z. B. als Shampoos, Geschirrspülmittel oder Haushaltsreinigungsmittel verwendet werden können. Alkylglycoside mit höherem Oligomerisierungsgrad weisen bessere hautpflegende Eigenschaften auf und können in reinigenden und hautpflegenden Kosmetika Einsatz finden.

Die Extraktion kann diskontinuierlich oder kontinuierlich durchgeführt werden. Bevorzugt ist aus wirtschaftlichen Gründen die kontinuierliche Extraktion im Gegenstrom.

Als Alkylglycoside, welche durch das erfindungsgemäße Verfahren aufgetrennt werden sollen, kommen insbesondere Verbindungen der allgemeinen Formel

$$R^1\text{-}R^2\text{-}O\text{-}G_a$$

in Betracht. Dabei haben die Reste folgende Bedeutung:

$R^1 =$     Kohlenwasserstoffrest mit 3 bis 21 C-Atomen,
$R^2 =$     $CH_2$, $(OC_nH_{2n})_m$, wobei

        n einen durchschnittlichen Zahlenwert von 2 bis 4, vorzugsweise 2 bis 3, und
        m einen durchschnittlichen Zahlenwert von 1 bis 30, vorzugsweise 1 bis 10, hat,

$G =$     ein von einer Aldose oder Ketose mit 5 oder 6 Kohlestoffatomen, bevorzugt von Glucose, abgeleiteter Rest,
$a =$     durchschnittlicher Zahlenwert von 1 bis 10, vorzugsweise 1 bis 6.

Anhand einer schematischen Darstellung sei eine Ausführungsform des erfindungsgemäßen Verfahrens am Beispiel der Alkylglucoside näher erläutert: Die Apparatur besteht aus zwei Kolonnen, von denen die eine, die Extraktionskolonne, zur Abtrennung der lipophileren Anteile, z. B. der Monoglucoside aus dem Glucosidgemisch dient. Die Monoglucoside werden als Extrakt erhalten. Die zweite Kolonne, die Regenerierkolonne, dient zur Abtrennung der extrahierten Komponenten aus dem Extraktionsmittel. Das Glucosidgemisch, das zuvor von überschüssigen freien Fettalkoholen befreit wurde, wird im mittleren Teil der Extraktionskolonne zugeführt. Das zirkulierende Extraktionsmittel belädt sich in der Extraktionskolonne bevorzugt mit Monoglucosiden. Die restlichen freien Fettalkohole lösen sich ebenfalls im Extraktionsmittel auf. Gleichzeitig löst sich das Extraktionsmittel in der nach unten strömenden Flüssigphase auf, in der sich die höheren Glucoside anreichern.

Um die Entstehung von Einphasigkeit in der Extraktionskolonne infolge der Anreicherung von freien Fettalkoholen zu verhindern, ist es unter Umständen zweckmäßig, die Temperatur zum Kolonnenkopf hin zu erhöhen. Bei geringen Restgehalten (< 2 %) des Einsatzgemisches an freien Fettalkoholen ist das jedoch nicht erforderlich.

Das Raffinat, das am Sumpf der Extraktionskolonne abgezogen wird, enthält unter dem Extraktionsdruck soviel an Extraktionsmittel gelöst, daß es niedrigviskos ist und keine Verstopfungen in Leitungen und Ventilen eintreten. Zweckmäßigerweise wird es in einer Sprühdüse unter Entspannung auf Umgebungsdruck versprüht. Dabei verdampft das Extraktionsmittel und kühlt das Produkt soweit ab, daß ein festes Pulver entsteht.

Das mit den leichter löslichen Komponenten, den freien Alkoholen und Monoglucosiden, beladene Extraktionsmittel verläßt den Verstärkerteil der Extraktionskolonne (EK) am Kopf. Das beladene Extraktionsmittel wird über ein Druckreduzierventil auf den Druck in der Regenerierkolonne entspannt und nach Durchlaufen eines Wärmetauschers der Regenerierkolonne (RK) im mittleren Teil zugeführt. In der Regenerierkolonne werden Druck und Temperatur so gewählt, daß das Extraktionsmittel eine Dichte von weniger als 150 kg/m$^3$ besitzt. Die Regenerierkolonne wird als Rektifikationskolonne betrieben. Das die Regenerierkolonne am Kopf verlassende Extraktionsmittel ist frei von den gelösten Komponenten. Die Rektifikation erfolgt bei einem Druck, der dem Dampfdruck des Extraktionsmittels bei etwa 25 bis 30 °C entspricht. Das dampfförmige Extraktionsmittel wird in einem Wärmetauscher abgekühlt und kondensiert. Das flüssige Extraktionsmittel wird zur Extraktionskolonne zurückgepumpt und vor Eintritt in die Extraktionskolonne in einem Wärmetauscher auf die Extraktionstemperatur erwärmt. Auf diese Weise wird die aufwendige Rekompression von gasförmigen Komponenten vermieden.

Am Boden der Regenerierkolonne wird das Extrakt abgezogen. Ein kleiner Teil wird als Rücklauf auf den Kopf der Extraktionskolonne zurückgepumpt und der Rest in einer Düse unter Entspannung auf Umgebungsdruck versprüht, wobei das restliche, im Extrakt gelöste Extraktionsmittel verdampft. Das Extrakt kühlt dabei ab und fällt als Pulver in fester Form an.

Für die Ausrüstung der Kolonnen sind im Prinzip die bei der Flüssig-Flüssig-Extraktion und der Rektifikationstechnik üblichen Packungen wie Raschigringe, Drahtgewebepackungen, Berlsättel, Drahtwendeln usw. geeignet. Eine Ausrüstung der Kolonnen mit Siebböden ist ebenfalls möglich.

An den folgenden Beispielen sei das Verfahren weiter erläutert.

Beispiel 1

250 g eines (C$_{12}$-C$_{14}$)-Alkylglucosidgemisches, das nach Analyse (Silylierung und Hochtemperaturgaschromatographie) folgende Zusammensetzung hat:

| Dodecanol | 1,9 % |
|---|---|
| Tetradecanol | 3,0 % |
| Hexadecanol | 0,4 % |
| Monoglucosid | 70,0 % |
| Diglucosid | 15,5 % |
| Triglucosid | 5,3 % |
| Tetraglucosid | 1,7 % |
| Pentaglucosid | 0,7 % |
| Glucose | 1,5 % |

und 130 g Ethanol werden in einen elektrisch beheizten 1-l-Schüttelautoklaven gegeben und auf 105 °C aufgeheizt. Dann werden etwa 250 g Butan zugepumpt und durch Schütteln des Autoklaven für eine gute Durchmischung gesorgt, bis sich Gleichgewicht eingestellt hat. Der Druck im Autoklaven beträgt etwa 23·10$^5$ Pa (23 bar). Nach Ruhestellung des Autoklaven trennen sich rasch eine butanreiche (im folgenden als gasförmige Phase bezeichnet) und eine flüssige Phase ab. (Butan befindet sich bei den genannten Bedingungen unterhalb seiner kritischen Temperatur und oberhalb seines Dampfdruckes, also im flüssigen Zustand. Es entstehen demnach zwei flüssige Phasen unterschiedlicher Zusammensetzung.). Mit Hilfe einer Kapillare mit einem Innendurchmesser von 0,3 mm werden aus beiden Phasen Proben entnommen und analysiert. Die Zusammensetzung des gelösten schwerflüchtigen Anteils in den beiden Phasen wird nach Abdampfen des Butans und des Ethanols durch Hochtemperaturgaschromatographie bestimmt.

Zusammensetzung der Gasphase abzüglich Ethanol und Butan:

| Fettalkohol | 19,2 % |
|---|---|
| Monoglucosid | 69,9 % |
| Diglucosid | 7,7 % |
| Triglucosid | 1,7 % |
| Tetraglucosid | 1,3 % |

Zusammensetzung der Flüssigphase abzüglich Ethanol und Butan:

| Fettalkohol | 2,7 % |
|---|---|

| (fortgesetzt) | |
|---|---|
| Monoglucosid | 68,8 % |
| Diglucosid | 17,5 % |
| Triglucosid | 6,5 % |
| Tetraglucosid | 3,3 % |

Der Trennfaktor Mono- / Diglucosid beträgt 2,4. In der gasförmigen Phase sind 5,8 % an schwerflüchtigen Bestandteilen gelöst; in der Flüssigphase 60 %. Die gasförmige Phase enthält 16 % Ethanol und 78 % Butan, die Flüssigphase 12 % Ethanol und 28 % Butan.

Beispiel 2

250 g eines $(C_{12}-C_{14})$-Alkylglucosidgemisches, das nach Analyse (Silylierung und Hochtemperaturgaschromatographie) folgende Zusammensetzung besitzt:

| Fettalkohol | 5,3 % |
|---|---|
| Monoglucosid | 70,0 % |
| Diglucosid | 15,5 % |
| Triglucosid | 5,3 % |
| Tetraglucosid | 1,7 % |
| Pentaglucosid | 0,7 % |
| Glucose | 1,5 % |

werden in einen elektrisch beheizten 1-l-Schüttelautoklaven gegeben und auf 70 °C aufgeheizt. Dann werden etwa 200 g Dimethylether und anschließend Kohlendioxid bis zu einem Druck von $70 \cdot 10^5$ Pa (70 bar) zugepumpt. Das Gemisch aus Kohlendioxid und Dimethylether ist unter diesen Bedingungen einphasig und wirkt als einheitliches Extraktionsmittel. Durch Schütteln des Autoklaven wird für gute Durchmischung gesorgt. Nach Ruhestellung des Autoklaven setzen sich rasch eine gasförmige und eine flüssige Phase ab. Mit Hilfe einer Kapillare mit einem Durchmesser von 0,3 mm werden aus beiden Phasen Proben entnommen und analysiert. Nach Abdampfen des Kohlendioxids und des Dimethylethers wird die Zusammensetzung des schwerflüchtigen Anteils der beiden Phasen durch Hochtemperaturgaschromatographie bestimmt. Die Beladung der Gasphase beträgt 3,2 %.

Die Gasphase hat nach Abzug von Kohlendioxid und Dimethylether folgende Zusammensetzung:

| Fettalkohol | 39,4 % |
|---|---|
| Monoglucosid | 54,4 % |
| Diglucosid | 3,6 % |
| Triglucosid | 0,3 % |

Die Zusammensetzung der Flüssigphase abzüglich Kohlendioxid und Dimethylether ist:

| Fettalkohol | 2,9 % |
|---|---|
| Monoglucosid | 71,1 % |
| Diglucosid | 15,7 % |
| Triglucosid | 5,0 % |
| Tetraglucosid | 3,5 % |
| Pentaglucosid | 0,5 % |
| Glucose | 1,8 % |

Das Extraktionsmittelgemisch in der gasförmigen Phase besteht aus 25 % Kohlendioxid und 75 % Dimethylether. Die Flüssigphase enthält 30 % Dimethylether und 7 % Kohlendioxid.

Beispiel 3

250 g eines $(C_{12}-C_{14})$-Alkylglucosidgemisches, das nach Analyse (Silylierung und Hochtemperaturgaschromatographie) folgende Zusammensetzung hat:

| Fettalkohol | 3,2 % |
|---|---|
| Monoglucosid | 71,6 % |
| Diglucosid | 15,8 % |
| Triglucosid | 5,4 % |
| Tetraglucosid | 1,7 % |
| Pentaglucosid | 0,7 % |
| Glucose | 1,5 % |

und 70 g Dimethylether werden in einen elektrisch beheizten 1 l Schüttelautoklaven gegeben und auf 60 °C aufgeheizt. Dann werden etwa 270 g Propan zugepumpt und durch Schütteln des Autoklaven für eine gute Durchmischung gesorgt, bis sich Gleichgewicht eingestellt hat. Der Druck im Autoklaven beträgt dabei etwa $50 \cdot 10^5$ Pa (50 bar). Nach Ruhestellung des Autoklaven trennen sich rasch eine propanreiche (im folgenden als gasförmige Phase bezeichnet) und eine flüssige Phase ab. Propan befindet sich bei der genannten Temperatur unterhalb seiner kritischen Temperatur; sein Dampfdruck ist geringer als der Prozessdruck. Demnach sind zwei flüssige Phasen verschiedener Zusammensetzung im Gleichgewicht vorhanden. Mit Hilfe einer Kapillare mit einem Innendurchmesser von 0,3 mm werden aus beiden Phasen Proben entnommen und analysiert. Die Zusammensetzung des schwerflüchtigen Anteils in den beiden Phasen wird nach Abdampfen des Propans und des Dimethylethers durch Silylierung mit nachfolgender Hochtemperaturgaschromatographie bestimmt. Die Beladung der Gasphase mit schwerflüchtigen Anteilen beträgt 7 Gew.%.

Zusammensetzung der Gasphase abzüglich Dimethylether und Propan:

| Fettalkohol | 12,5 % |
|---|---|

(fortgesetzt)

| | |
|---|---|
| Monoglucosid | 75,4 % |
| Diglucosid | 8,0 % |
| Triglucosid | 1,8 % |
| Tetraglucosid | 1,3 % |

Zusammensetzung der Flüssigphase abzüglich Dimethylether und Propan:

| | |
|---|---|
| Fettalkohol | 2,4 % |
| Monoglucosid | 71,2 % |
| Diglucosid | 16,5 % |
| Triglucosid | 5,7 % |
| Tetraglucosid | 1,7 % |
| Pentaglucosid | 0,1 % |
| Glucose | 1,7 % |

Der Trennfaktor Mono- / Diglucosid beträgt 2,2. In der gasförmigen Phase sind 7 % an schwerflüchtigen Bestandteilen gelöst; in der flüssigen Phase 60 %. Die gasförmige Phase enthält Dimethylether und Propan im Verhältnis von 1 zu 4; in der Flüssigphase sind Dimethylether und Propan im Verhältnis 45 zu 55 gelöst.

Beispiel 4

Ein ($C_{12}$-$C_{14}$)-Alkylglucosidgemisch der folgenden Zusammensetzung:

| | |
|---|---|
| Freie Fettalkohole | 5,3 % |
| Monoglucoside | 70,0 % |
| Diglucoside | 15,5 % |
| Triglucoside | 5,3 % |
| Tetraglucoside | 1,7 % |
| Pentaglucoside | 0,7 % |
| Glucose | 1,5 % |

wird in einem Druckbehälter mit 15 % Dimethylether versetzt, dabei entsteht eine pumpbare Flüssigkeit. Die erhaltene flüssige Phase wird der Extraktionskolonne einer Anlage gemäß Abb. 1 im mittleren Bereich in einer Menge von 2 kg/h zugepumpt. Auf dem Weg von der Pumpe zur Kolonne wird die Temperatur des Gemisches in einem Wärmetauscher auf 60 °C eingestellt. Die 4 m hohe Extraktionskolonne ist mit einer Packung Typ Sulzer CY ausgerüstet. In der Extraktionskolonne wird die flüssige Lösung von Dimethylether im Alkylglucosid im Gegenstrom mit einem Extraktionsmittel, welches aus einem Gemisch aus 80 % Propan und 20 % Dimethylether besteht, bei einem Druck von 50·$10^5$ Pa (50 bar) und einer Temperatur von 60 °C in Kontakt gebracht. Das flüssige Alkylglucosid-Gemisch fließt wegen seines höheren spezifischen Gewichtes in der Kolonne nach unten. Das Extraktionsmittel durchströmt die Kolonne von unten nach oben. Dabei gehen bevorzugt Monoglucoside und freie Fettalkohole in Lösung. Das Extraktionsmittel verläßt die Extraktionskolonne am Kopf mit einer Beladung von 7 % und wird der Regenerierkolonne im mittleren Bereich zugeführt.

In der Regenerierkolonne werden Extraktionsmittel und Extrakt durch Rektifikation voneinander getrennt. Die Rektifikation wird bei einem Druck von 12·$10^5$ Pa (12 bar) durchgeführt. Die Temperatur am Sumpf der Regenerierkolonne beträgt 90 °C. Das Extrakt, welches am Sumpf der Regenerierkolonne abgezogen wird, enthält noch soviel an Dimethylether und Propan gelöst, daß das Gemisch als Flüssigkeit vorliegt. Ein Teil des Extraktes wird als Rücklauf auf den Kopf der Extraktionskolonne zurückgeführt. Bei der Entspannung des als Produkt entnommenen Extraktes auf Umgebungsdruck wird das Extrakt versprüht. Dabei verdampfen Dimethylether und Propan und das Extrakt fällt als Pulver an.

Bei einem Lösemittelverhältnis (solvent to feed ratio) von 9 fallen 63 % des Zulaufs als Extrakt der folgenden Zusammensetzung an:

| | |
|---|---|
| Freie Fettalkohole | 8,4 % |
| Monoglucoside | 86,0 % |
| Diglucoside | 5,4 % |
| Triglucoside | 0,1 % |

Das Raffinat, welches 37 % des Zulaufes ausmacht, hat folgende Zusammensetzung:

| | |
|---|---|
| Freie Fettalkohole | - |
| Monoglucoside | 42,7 % |
| Diglucoside | 32,7 % |
| Triglucoside | 14,1 % |
| Tetraglucoside | 4,6 % |
| Pentaglucoside | 1,9 % |
| Glucose | 4,1 % |

Beispiel 5

Ein Alkylglucosidgemisch mit einem hohen Gehalt an Stearylpolyglucosiden hat die folgende Zusammensetzung:

| | |
|---|---|
| Freie Fettalkohole | 1,2 % |
| Monoglucoside | 72,8 % |
| Diglucoside | 16,2 % |
| Triglucoside | 5,5 % |
| Tetraglucoside | 1,8 % |
| Pentaglucoside | 0,7 % |
| Glucose | 1,8 % |

Das Gemisch wird in einem Druckbehälter mit 20 % Dimethylether (im folgenden mit DME bezeichnet) bei Umgebungstemperatur versetzt. Dabei entsteht eine pumpbare Flüssigkeit. Die erhaltene flüssige Phase wird der Extraktionskolonne einer Anlage gemäß Abb. 1 im mittleren Bereich in einer Menge von etwa 1,0 kg/ h zugepumpt. Auf dem Weg von der Pumpe zur Kolonne wird das Gemisch in einem Wärmetauscher auf 140 °C erwärmt. Die 4 m hohe Extraktionskolonne ist mit einer Packung Typ Sulzer CY ausgerüstet. In der Extraktionskolonne wird das flüssige Gemisch aus Alkylglucosid und DME im Gegenstrom mit DME als Extraktionsmittel bei einem Druck von $60 \cdot 10^5$ Pa (60 bar) und einer Temperatur von 140 °C in Kontakt gebracht. Das flüssige Alkylglucosid-Gemisch fließt wegen seines hohen spezifischen Gewichtes in der Kolonne nach unten. Das Extraktionsmittel durchströmt die Kolonne von unten nach oben. Dabei gehen bevorzugt Monoglucoside und freie Fettalkohole in Lösung. Das Extraktionsmittel verläßt die Extraktionskolonne am Kopf mit einer Beladung von etwa 6 % und wird der Regenerierkolonne im mittleren Bereich zugeführt.

In der Regenerierkolonne werden Extraktionsmittel und Extrakt durch Verminderung des Druckes auf $30 \cdot 10^5$ Pa (30 bar) voneinander getrennt. Dabei kühlt sich die Temperatur infolge der Entlösung des Extraktionsmittels ab. Das Extrakt, welches am Sumpf der Regenerierkolonne anfällt, enthält noch soviel DME gelöst, daß es in flüssigem Zustand vorliegt. Ein Teil des Extraktes wird als Rücklauf auf den Kopf der Extraktionskolonne zurückgeführt. Bei der Entspannung der Hauptmenge des Extraktes auf Umgebungsdruck wird das Extrakt versprüht. Dabei verdampft der gelöste Dimethylether und das Extrakt fällt als Pulver an.

Das die Regenerierkolonne verlassende Extraktionsmittel wird auf etwa 40 °C abgekühlt und kondensiert. Der flüssige DME wird auf Extraktionsdruck gebracht, anschließend in einem Wärmetauscher auf 140 °C erwärmt und in die Extraktionskolonne zurückgeführt.

Bei einem Lösemittelverhältnis (solvent to feed ratio) von etwa 15 fallen 79,8 % des Zulaufes als Extrakt der folgenden Zusammensetzung (nach Abzug des gelösten Extraktionsmittels) an:

| Freie Fettalkohole | 1,4 % |
|---|---|
| Monoglucoside | 89,5 % |
| Diglucoside | 7,3 % |
| Triglucoside | 1,1 % |
| Tetraglucoside | 0,4 % |

Das Raffinat, welches 20,2 % des Zulaufes ausmacht, hat folgende Zusammensetzung (nach Abzug des Extraktionsmittels) :

| Freie Fettalkohole | - |
|---|---|
| Monoglucoside | 5,9 % |

(fortgesetzt)

| Diglucoside | 51,5 % |
|---|---|
| Triglucoside | 22,8 % |
| Tetraglucoside | 7,4 % |
| Pentaglucoside | 3,5 % |
| Glucose | 8,9 % |

Vergleichsbeispiel 6

Ein Alkylglucosidgemisch mit hohem Gehalt an Stearylglucosiden hat folgende Zusammensetzung:

| Freie Fettalkohole | 74,4 % |
|---|---|
| Monoglucoside | 20,3 % |
| Diglucoside | 3,4 % |
| Triglucoside | 1,1 % |
| Tetraglucoside | 0,7 % |
| Pentaglucoside | - |
| Glucose | 0,9 % |

Das Gemisch wird der Extraktionskolonne einer Anlage gemäß Abb. 1 im mittleren Bereich in einer Menge von etwa 1,8 kg/h zugepumpt. Auf dem Weg von der Pumpe zur Kolonne wird das Alkylglucosid-Gemisch in einem Wärmetauscher auf 120 °C erwärmt. Die 4 m hohe Extraktionskolonne ist mit einer Packung Typ Sulzer CY ausgerüstet. In der Extraktionskolonne wird das flüssige Gemisch aus Alkylglucosid und Fettalkoholen im Gegenstrom mit einem Gemisch aus 90 Gew.% Propan und 10 Gew.% Dimethylether als Extraktionsmittel bei einem Druck von $70 \cdot 10^5$ Pa (70 bar) und einer Temperatur von 120 °C in Kontakt gebracht.

Das Gemisch aus Alkylglucosid und freien Fettalkoholen durchströmt wegen seiner höheren Dichte die Kolonne von oben nach unten. Das Propan durchströmt im Gegenstrom die Kolonne von unten nach oben. Dabei gehen bevorzugt die freien Fettalkohole und mit ihnen ein Teil der Monoglucoside in Lösung. Wird ohne Rückfluß extrahiert, so entsteht ein Extrakt, welches noch größere Anteile an Alkylglucosid enthalten kann und beispielsweise folgende Zusammensetzung hat:

| Freie Fettalkohole | 86,4 % |
|---|---|
| Monoglucoside | 12,9 % |
| Diglucoside | 1,0 % |
| Triglucoside | 0,3 % |
| Tetraglucoside | 0,1 % |
| Pentaglucoside | n. n. |

Die Höhe des Glucosidgehaltes im Extrakt kann durch Änderung des Temperaturgefälles in der Extraktionskolonne in gewissen Grenzen variiert werden.

Das Raffinat, das 14,1 % des Zulaufes ausmacht, hat abzüglich des Extraktionsmittels folgende Zusammensetzung:

| Freie Fettalkohole | 1,6 % |
|---|---|
| Monoglucoside | 65,2 % |
| Diglucoside | 17,7 % |
| Triglucoside | 5,7 % |
| Tetraglucoside | 4,3 % |
| Glucose | 6,4 % |

In einer zweiten Extraktionsstufe werden sodann die freien Fettalkohole des Extraktes von den Glucosiden getrennt. Dabei dient wiederum Propan als Extraktionsmittel. Das Extrakt der ersten Stufe wird der Extraktionskolonne einer Anlage gemäß Abb. 1 im mittleren Bereich in einer Menge von etwa 1,8 kg/h zugepumpt. Auf dem Weg zur Kolonne wird das Extrakt in einem Wärmetauscher auf 120 °C erwärmt. Die 4 m hohe Extraktionskolonne ist mit einer Packung Typ Sulzer CY ausgerüstet. In der Extraktionskolonne wird das flüssige Extrakt im Gegenstrom mit Propan als Extraktionsmittel bei einem Druck von $70 \cdot 10^5$ Pa (70 bar) und einer Temperatur von 120 °C in Kontakt gebracht. Die Temperatur in der Extraktionskolonne nimmt vom Sumpf der Kolonne von 120 °C bis zum Kopf auf 140 °C zu.

Durch die Erhöhung der Temperatur zum Kopf der Extraktionskolonne wird die Löslichkeit der Alkylglucoside im dichten Propan soweit herabgesetzt, daß die Verluste an Alkylglucosiden in Verbindung mit dem Austrag der Fettalkohole gering sind (weniger als 3 %). Gleichzeitig wird ein Teil des Extraktes als Rücklauf auf den Kopf der Extraktionskolonne zurückgeführt, um die im Extrakt der Stufe 1 befindlichen Alkylglucoside vollständig im Raffinat der zweiten Stufe zu gewinnen. Das Extrakt der zweiten Stufe hat folgende Zusammensetzung:

| Freie Fettalkohole | 99,5 % |
|---|---|
| Monoglucoside | 0,4 % |
| Diglucoside | 0,1 % |
| Triglucoside | n. n. |
| Tetraglucoside | n. n. |

Das Raffinat, das 14,2 % des Zulaufs ausmacht, hat abzüglich des Extraktionsmittels folgende Zusammensetzung:

| Freie Fettalkohole | 2,1 % |
|---|---|
| Monoglucoside | 88,7 % |
| Diglucoside | 6,3 % |
| Triglucoside | 2,1 % |
| Tetraglucoside | 0,7 % |

**Patentansprüche**

1.  Verfahren zur Auftrennung von Alkylglycosidgemischen, bei denen der aus der Synthese stammende überschüssige Alkohol zuvor abgetrennt wurde, in Fraktionen unterschiedlichen Oligomerisierungsgrads durch Extraktion mit einem nahekritischen oder überkritischen Extraktionsmittel, das sich im Bereich der reduzierten Temperatur von 0,7 bis 1,3 befindet, und bei Drücken, die so gewählt werden, daß die Dichte des Extraktionsmittels ≥ 180 kg/m³ beträgt.

2.  Verfahren nach Anspruch 1, dadurch gekennzeichnet, daß als nahekritisches Extraktionsmittel Kohlendioxid oder ein Kohlenwasserstoff, der 1 bis 10 Kohlenstoffatome aufweist, oder Gemische aus diesen verwendet werden.

3.  Verfahren nach Anspruch 1, dadurch gekennzeichnet, daß als nahekritisches Extraktionsmittel ein Ether mit 2 bis 8 Kohlenstoffatomen verwendet wird.

4.  Verfahren nach Anspruch 1 und 3, dadurch gekennzeichnet, daß als nahekritisches Extraktionsmittel Dimethylether verwendet wird.

5.  Verfahren nach Anspruch 1, 3 und 4, dadurch gekennzeichnet, daß als nahekritisches Extraktionsmittel ein Gemisch aus Dimethylether und Kohlendioxid verwendet wird.

6.  Verfahren nach Anspruch 4, dadurch gekennzeichnet, daß als nahekritisches Extraktionsmittel ein Gemisch aus Dimethylether und Propan verwendet wird.

7.  Verfahren nach Anspruch 4, dadurch gekennzeichnet, daß als nahekritisches Extraktionsmittel ein Gemisch aus Dimethylether und Butan verwendet wird.

8.  Verfahren nach Anspruch 1 und 3, dadurch gekennzeichnet, daß als nahekritisches Extraktionsmittel Ethylmethylether verwendet wird.

9.  Verfahren nach Anspruch 1 und 2, dadurch gekennzeichnet, daß als nahekritisches Extraktionsmittel Propan oder Butan oder deren Mischungen verwendet werden.

10. Verfahren nach den Ansprüchen 1 bis 9, dadurch gekennzeichnet, daß die Extraktion bei einem Druck durchgeführt wird, der bis $80 \cdot 10^5$ Pa, vorzugsweise $10 \cdot 10^5$ Pa bis $60 \cdot 10^5$ Pa, höher ist als der Dampfdruck des Extraktionsmittels über der Lösung.

**11.** Verfahren nach den Ansprüchen 1 bis 10, dadurch gekennzeichnet, daß die Extraktion mehrstufig im Gegenstrom durchgeführt wird.

**12.** Verfahren nach den Ansprüchen 1 bis 11, dadurch gekennzeichnet, daß das Extrakt, welches am Sumpf der Regenerierkolonne abgezogen wird, über eine Sprühdüse entspannt und das Produkt in Form eines Pulvers erhalten wird.

**13.** Verfahren nach Anspruch 1 bis 12, dadurch gekennzeichnet, daß die Regenerierung des Extraktionsmittels durch Rektifikation bei einem Druck erfolgt, der dem Dampfdruck des Extraktionsmittels bei 25 bis 30 °C entspricht oder geringfügig darüber liegt, und das den Kopf der Regenerierkolonne in gasförmigem Zustand verlassende Extraktionsmittel gekühlt, kondensiert und in flüssigem Zustand in die Extraktionskolonne zurückgepumpt wird.

**14.** Verfahren nach den Ansprüchen 1 bis 13, dadurch gekennzeichnet, daß den nahekritischen Extraktionsmitteln bis zu 35 Gew.-% aliphatische Alkohole, Ketone oder Säuren, insbesondere Ethanol, und/oder Wasser zugesetzt werden.

**15.** Verfahren nach den Ansprüchen 1 bis 14, dadurch gekennzeichnet, daß man in einem ersten Extraktionsschritt das Alkylmonoglycosid vollständig extrahiert und die verbleibenden Alkylpolyglycoside durch eine zweite Fraktionierung in eine reine Alkylglycosid-reiche Extraktphase und eine die Nebenprodukte und Verunreinigungen enthaltende Phase auftrennt.

**16.** Verfahren nach den Ansprüchen 1 bis 14, dadurch gekennzeichnet, daß man eine Alkylglycosid-reiche Extraktphase von den farbigen Verunreinigungen abtrennt.

**17.** Verfahren nach Anspruch 16, dadurch gekennzeichnet, daß man in einem ersten Schritt eine Alkylglycosid-reiche Extraktphase von den farbigen Verunreinigungen abtrennt und aus der Extraktphase in weiteren Extraktionsschritten das Alkylmonoglycosid abtrennt.

**Claims**

**1.** Process for separating alkyl glycoside mixtures in which the excess alcohol originating from the synthesis has been separated off in advance into fractions of differing degree of oligomerization by extraction with a near-critical or supercritical extraction medium which is in the reduced temperature range of from 0.7 to 1.3 and at pressures which are chosen so that the density of the extraction medium is $\geq$ 180 kg/m$^3$.

**2.** Process according to Claim 1, characterized in that, as near-critical extraction medium, use is made of carbon dioxide or a hydrocarbon which has from 1 to 10 carbon atoms or mixtures of these.

**3.** Process according to Claim 1, characterized in that, as near-critical extraction medium, use is made of an ether having from 2 to 8 carbon atoms.

**4.** Process according to Claim 1 and 3, characterized in that, as near-critical extraction medium, use is made of dimethyl ether.

**5.** Process according to Claim 1, 3 and 4, characterized in that, as near-critical extraction medium, use is made of a mixture of dimethyl ether and carbon dioxide.

**6.** Process according to Claim 4, characterized in that, as near-critical extraction medium, use is made of a mixture of dimethyl ether and propane.

**7.** Process according to Claim 4, characterized in that, as near-critical extraction medium, use is made of a mixture of dimethyl ether and butane.

**8.** Process according to Claim 1 and 3, characterized in that, as near-critical extraction medium, use is made of ethyl methyl ether.

**9.** Process according to Claim 1 and 2, characterized in that, as near-critical extraction medium, use is made of propane or butane or their mixtures.

**10.** Process according to Claims 1 to 9, characterized in that the extraction is carried out at a pressure which is higher by up to $80 \times 10^5$ Pa, preferably by from $10 \times 10^5$ Pa to $60 \times 10^5$ Pa, than the vapour pressure of the extraction medium above the solution.

**11.** Process according to Claims 1 to 10, characterized in that the extraction is carried out in a multistage manner in countercurrent.

**12.** Process according to Claims 1 to 11, characterized in that the extract which is taken off at the bottom of the regeneration column is expanded via a spray nozzle and the product is obtained in the form of a powder.

**13.** Process according to Claim 1 to 12, characterized in that the extraction medium is regenerated by rectification at a pressure which corresponds to the vapour pressure of the extraction medium at from 25 to 30°C or is slightly above this, and the extraction medium leaving the top of the regeneration column in the gaseous state is cooled, condensed and, in the liquid state, pumped back to the extraction column.

14. Process according to Claims 1 to 13, characterized in that up to 35% by weight of aliphatic alcohols, ketones or acids, in particular ethanol, and/or water are added to the near-critical extraction media.

15. Process according to Claims 1 to 14, characterized in that, in a first extraction step, the alkyl monoglycoside is completely extracted and the remaining alkyl polyglycosides are separated by a second fractionation into a pure alkyl-glycoside-rich extract phase and a phase comprising the byproducts and contaminants.

16. Process according to Claims 1 to 14, characterized in that an alkyl-glycoside-rich extract phase is separated off from the coloured contaminants.

17. Process according to Claim 16, characterized in that, in a first step, an alkyl-glycoside-rich extract phase is separated off from the coloured contaminants and, in further extraction steps, the alkyl monoglycoside is separated off from the extract phase.

## Revendications

1. Procédé de séparation de mélanges d'alkylglycosides, dans lesquels l'alcool en excès provenant de la synthèse est séparé préalablement, en fractions de différents degrés d'oligomérisation, par extraction avec un agent d'extraction quasi critique ou supercritique, qui se situe dans l'intervalle de température réduite de 0,7 à 1,3, et sous des pressions qui sont choisies de telle sorte que la densité de l'agent d'extraction s'élève à $\geq$ 180 kg/m$^3$.

2. Procédé suivant la revendication 1, caractérisé en ce qu'on utilise comme agent d'extraction quasi critique du dioxyde de carbone ou un hydrocarbure, qui présente de 1 à 10 atomes de carbone, ou des mélanges de ceux-ci.

3. Procédé suivant la revendication 1, caractérisé en ce qu'on utilise comme agent d'extraction quasi critique un éther avec 2 à 8 atomes de carbone.

4. Procédé suivant les revendications 1 et 3, caractérisé en ce qu'on utilise comme agent d'extraction quasi critique du diméthyléther.

5. Procédé suivant les revendications 1, 3 et 4, caractérisé en ce qu'on utilise comme agent d'extraction quasi critique un mélange de diméthyléther et de dioxyde de carbone.

6. Procédé suivant la revendication 4, caractérisé en ce qu'on utilise comme agent d'extraction quasi critique un mélange de diméthyléther et de propane.

7. Procédé suivant la revendication 4, caractérisé en ce qu'on utilise comme agent d'extraction quasi critique un mélange de diméthyléther et de butane.

8. Procédé suivant les revendications 1 et 3, caractérisé en ce qu'on utilise comme agent d'extraction quasi critique de l'éthylméthyléther.

9. Procédé suivant les revendications 1 et 2, caractérisé en ce qu'on utilise comme agent d'extraction quasi critique du propane ou du butane ou leurs mélanges.

10. Procédé suivant les revendications 1 à 9, caractérisé en ce que l'extraction est réalisée à une pression qui est de jusqu'à 80•10$^5$ Pa, de préférence de 10•10$^5$ Pa à 60•10$^5$ Pa, supérieure à la pression de vapeur de l'agent d'extraction au-dessus de la solution.

11. Procédé suivant les revendications 1 à 10, caractérisé en ce que l'extraction est réalisée à contre-courant en plusieurs étapes.

12. Procédé suivant les revendications 1 à 11, caractérisé en ce que l'extrait, lequel est soutiré au bas de la colonne de régénération, est détendu par une buse de pulvérisation, et en ce qu'on obtient le produit sous la forme d'une poudre.

13. Procédé suivant les revendications 1 à 12, caractérisé en ce que la régénération de l'agent d'extraction s'effectue par rectification à une pression qui correspond à la pression de vapeur de l'agent d'extraction entre 25 et 30°C ou qui est légèrement supérieure à celle-ci, et en ce que l'agent d'extraction quittant la tête de la colonne de régénération sous forme de gaz est refroidi, condensé et repompé à l'état liquide dans la colonne d'extraction.

14. Procédé suivant les revendications 1 à 13, caractérisé en ce qu'on ajoute aux agents d'extraction quasi critiques jusqu'à 35% en poids d'alcools, de cétones ou d'acides aliphatiques, en particulier de l'éthanol, et/ou de l'eau.

15. Procédé suivant les revendications 1 à 14, caractérisé en ce qu'on extrait complètement l'alkylmonoglycoside, dans une première étape d'extraction, et que l'on sépare les alkylpolyglycosides restants par un deuxième fractionnement en une phase d'extraction pure riche en alkylglycoside et en une phase contenant les sous-produits et les impuretés.

16. Procédé suivant les revendications 1 à 14, caractérisé en ce qu'on sépare une phase d'extraction riche en alkylglycoside des impuretés colorées.

17. Procédé suivant la revendication 16, caractérisé en ce qu'on sépare dans une première étape une phase d'extraction riche en alkylglycoside des impuretés colorées, et en ce qu'on sépare l'alkylmonoglycoside de la phase d'extraction dans d'autres étapes d'extraction.